# EUROPEAN PATENT SPECIFICATION

(11) **EP 3 643 226 B1**
(45) Date of publication and mention of the grant of the patent: **31.07.2024**
(21) Application number: 19160241.6
(22) Date of filing: 01.03.2019
(51) Int. Cl.: A61B 5/024, A61B 5/11, A61B 5/00

(54) **HEART RATE ESTIMATION TAKING MOBILITY STATES INTO ACCOUNT**
HERZFREQUENZBESTIMMUNG UNTER BERÜCKSICHTIGUNG VON BEWEGUNGSZUSTÄNDEN
ESTIMATION DE LA FRÉQUENCE CARDIAQUE PRENANT COMPTE DE L'ÉTAT DE MOUVEMENT

(30) Priority: 23.10.2018 IN 201821040008
(43) Date of publication of application: 29.04.2020
(73) Proprietor: Tata Consultancy Services Limited, Maharashtra (IN)
(72) Inventor: MUKHOPADHYAY, SHALINI, 700160 Kolkata - West Bengal (IN); GHOSE, AVIK, 700160 Kolkata - West Bengal (IN); AHMED, NASIMUDDIN, 700160 Kolkata - West Bengal (IN); PAL, ARPAN, 700160 Kolkata - West Bengal (IN)
(74) Representative: Goddar, Heinz J.

(56) References cited:
- EP-A1- 3 330 972
- US-A1- 2017 164 851
- US-A1- 2018 199 837
- US-B2- 9 848 823

## Description

### CROSS-REFERENCE TO RELATED APPLICATIONS AND PRIORITY

The present application claims priority to Indian complete specification (Title: SYSTEM AND METHOD FOR HEART RATE ESTIMATION USING A TRACKING MECHANISM) Application No. (201821040008), filed in India on October 23, 2018.

### TECHNICAL FIELD

The disclosure herein generally relates to heart rate monitoring and estimation, and, more particularly, to a system and a method for improving the heart rate estimation based on a determined transition state of the user.

### BACKGROUND

One important measurement performed by many of the health monitoring equipment(s) is heart rate measurement, as heart rate is a crucial health parameter which can be indicative of health status of a user being monitored. The heart rate is typically measured in beats per minute (BPM). Many electronic heart rate monitors (also referred to as 'monitors') for measuring heart rate are available in the market today. Such heart rate monitoring and estimation devices are available in different forms. Some popular forms are chest straps, and different types of (smart) wearable devices (examples include watches, rings, wristbands, chest straps, headbands, headphones, ear buds, clamps, clips, clothing, bags, shoes, glasses, goggles, hats, suits, necklace, attachments/patches/strips/pads which can adhere to a living being, accessories, portable devices, and so on).

Many of such monitors currently being used are automatic, which means these devices can monitor and estimate heart rate of the user without requiring user intervention. Some of such devices may also be configured to trigger certain actions when certain preset conditions are detected/met. For example, the condition detected maybe a sudden variation in heart rate, which could be indicative of a health issue of the user, and in that scenario, the action maybe triggering an alarm to notify the user, and/or any other person.

However, these heart rate monitors have certain disadvantage(s) that they are often not very accurate, due to a high amount of noise present in the signals provided by the sensors of these monitors. The noise may be caused due to various reasons. One such reason is the user being monitored involving in any physical activity. When the user is engaged in a physical activity such as walking, climbing, jogging and so on, such activities may cause heart rate of the user to rise, and it may be a normal process. However, a system that is configured to trigger an alarm in response to an abnormal variation in the heart rate may still trigger the alarm upon detecting the variation in the heart rate, even though it was caused due to the user involving in the physical activity, which means the system triggered a false alarm. Some of the existing systems handle such scenarios by monitoring and considering mobility states of the user at the time of measurement of the heart rate. The term 'mobility states' may refer to state of motion or state of rest. If detected mobility state of the user indicates that the user was in motion and/or was involved in any physical activity at a time an 'abnormal' spike in the value of heart rate of the user was detected, then the system may filter out the detected spike, which in turn eliminates chances of the system triggering a false alarm. However, such systems may fail to provide accurate results as the mobility state of a user may not be constant throughout the heart rate estimation. Document US 2017/164851 A1 discloses a system comprising a biological-information computing section configured to perform computation of biological information such as pulse wave information. The biological-information computing section is configured to perform noise reduction processing for reducing body motion noise. The noise reduction processing is performed differently depending on whether body motion information indicates a state S1, which is a rest state, a state S2, which is an exercise state in which motion with low periodicity or continuity is performed, or a state S3, which is an exercise state in which motion with high periodicity and continuity is performed.

### SUMMARY

The invention is defined by the appended claims. Embodiments of the present disclosure present technological improvements as solutions to one or more of the above-mentioned technical problems recognized by the inventors in conventional systems. In one embodiment, a system for heart rate estimation is provided and is as defined in independent claim 4. The system includes a memory module; one or more communication interfaces; a heart rate estimation module, a post processing module, and one or more hardware processors coupled to the memory module via the one or more communication interfaces, wherein the one or more hardware processors are caused by the plurality of instructions to: estimate heart rate data of a user, via the heart rate estimation module, wherein the estimated heart rate is distributed among a plurality of time windows; collect data pertaining to a first mobility state and a second mobility state of the user, while estimating the heart rate data; process the estimated heart rate data and the data pertaining to the first mobility state and the second mobility state, comprising: (a) identifying a transition state of the user as one of a first transition state, a second transition state, or a third transition state, in terms of transition between the first mobility state and the second mobility state at the time of estimating the heart rate data; (b) filtering the estimated heart rate data, via the post processing module, based on the identified transition state of the user; and (c) generating the filtered heart rate data as output.

In another aspect, a method for heart rate estimation is provided and is as defined in the independent claim 1. The method includes steps of: estimating heart rate data of a user, wherein the estimated heart rate is distributed among a plurality of time windows; collecting data pertaining to a first mobility state and a second mobility state of the user, while estimating the heart rate data; processing the estimated heart rate data and the data pertaining to the first mobility state and the second mobility state, comprising: (a) identifying a transition state of the user as one of a first transition state, a second transition state, or a third transition state, in terms of transition between the first mobility state and the second mobility state while estimating the heart rate data; (b) filtering the estimated heart rate data based on the identified transition state of the user; and (f) generating the filtered heart rate data as output.

In yet another aspect, a non-transitory computer readable medium for heart rate estimation is provided and is as defined in the independent claim 7. The non-transitory computer readable medium performs the heart rate estimation by: estimating heart rate data of a user, wherein the estimated heart rate is distributed among a plurality of time windows; collecting data pertaining to a first mobility state and a second mobility state of the user, while estimating the heart rate data; processing the estimated heart rate data and the data pertaining to the first mobility state and the second mobility state, comprising: (a) identifying a transition state of the user as one of a first transition state, a second transition state, or a third transition state, in terms of a transition between the first mobility state and the second mobility state while estimating the heart rate data; (b) filtering the estimated heart rate data based on the identified transition state of the user; and (f) generating the filtered heart rate data as output.

It is to be understood that both the foregoing general description and the following detailed description are exemplary and explanatory only and are not restrictive of the invention which is defined by the appended claims.

### BRIEF DESCRIPTION OF THE DRAWINGS

The accompanying drawings, which are incorporated in and constitute a part of this disclosure, illustrate exemplary embodiments and, together with the description, serve to explain the disclosed principles:
FIG. 1 illustrates an exemplary block diagram of a system for heart rate estimation, in accordance with some embodiments of the present disclosure.
FIG. 2 is a flow diagram depicting the steps involved in the process of heart rate estimation and filtering of estimated heart rate data using the system of FIG. 1, in accordance with some embodiments of the present disclosure.
FIG. 3 is a flow diagram depicting the steps involved in the process of filtering the estimated heart rate data by executing a first method using the system of FIG. 1, in accordance with some embodiments of the present disclosure.
FIG. 4 is a flow diagram depicting the steps involved in the process of filtering the estimated heart rate data by executing a second method using the system of FIG. 1, in accordance with some embodiments of the present disclosure.
FIG. 5 is a flow diagram depicting the steps involved in the process of filtering the estimated heart rate data by executing a third method using the system of FIG. 1, in accordance with some embodiments of the present disclosure.

### DETAILED DESCRIPTION

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts It is intended that the following detailed description be considered as exemplary only, with the true scope indicated by the following claims.

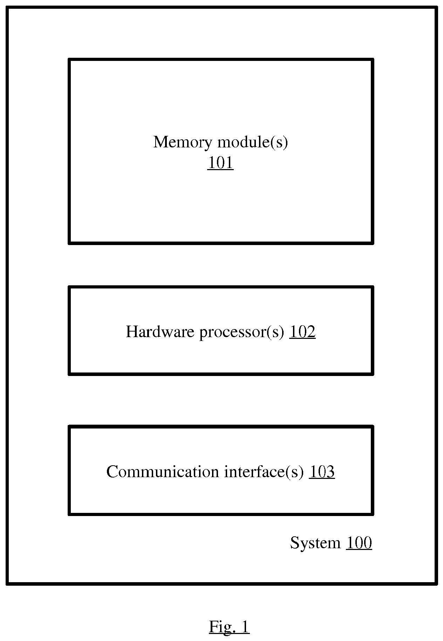

Referring now to the drawings, and more particularly to FIG. 1 through FIG. 5, where similar reference characters denote corresponding features consistently throughout the figures, there are shown preferred embodiments and these embodiments are described in the context of the following exemplary system and/or method.

FIG. 1 illustrates an exemplary block diagram of a system for heart rate estimation, according to some embodiments of the present disclosure. In an embodiment, the system 100 includes one or more hardware processors 102, communication interface(s) or input/output (I/O) interface(s) 103, and one or more data storage devices or memory module 101 operatively coupled to the one or more hardware processors 102. The one or more hardware processors 102 can be implemented as one or more microprocessors, microcomputers, microcontrollers, digital signal processors, central processing units, state machines, graphics controllers, logic circuitries, and/or any devices that manipulate signals based on operational instructions. Among other capabilities, the processor(s) are configured to fetch and execute computer-readable instructions stored in the memory. In an embodiment, the system 100 can be implemented in a variety of computing systems, such as laptop computers, notebooks, hand-held devices, workstations, mainframe computers, servers, a network cloud and the like.

The communication interface(s) 103 can include a variety of software and hardware interfaces, for example, a web interface, a graphical user interface, and the like and can facilitate multiple communications within a wide variety of networks N/W and protocol types, including wired networks, for example, LAN, cable, etc., and wireless networks, such as WLAN, cellular, or satellite. In an embodiment, the communication interface(s) 103 can include one or more ports for connecting a number of devices to one another or to another server.

The memory module(s) 101 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, one or more modules (not shown) of the system 100 can be stored in the memory 101.

The system 100, using the one or more hardware processors (also referred to as 'processors' throughout the specification) estimates heart rate of a user. The system 100 may use any of the existing heart rate estimation mechanisms for the purpose of estimating the heart rate. In another embodiment, the system 100 may be configured to track heart rate estimation being performed by any external system, and collect heart rate data estimated by the external system, as input, for further processing. For the purpose of processing the estimated heart rate data, the system 100 distributes the heart rate data into multiple time windows, in the order the heart rate data is estimated, and based on corresponding timestamp (matching the estimated heart rate data).

The system 100, along with the heart rate data, also collects data pertaining to at least two mobility states, of the user being monitored. For the purpose of explanation, two mobility states (a first mobility state and a second mobility state) are considered. The 'mobility states' are 'a state of rest' and a 'state of motion'. Again, for the purpose of explanation, the 'state of rest' is termed as a 'first mobility state' and the 'state of motion' is termed as a 'second mobility state'. It is to be noted that in an alternate embodiment, the state of motion maybe the first mobility state and the state of rest may be the second mobility state. The heart rate estimation process is explained in the specification by considering the 'state of rest' as the 'first mobility state' and the 'state of motion' as the 'second mobility state'.

Further, the system 100 considers heart rate data and mobility state data from two windows (a first window and a second window) of the plurality of time windows, at a time, for processing. During the processing, the system 100 identifies a transition state of the user between the first and second windows considered. In an embodiment, the system 100 identifies the transition state of the user, based on the data pertaining to the mobility states i.e. the transition state of the user represents/indicates presence or absence of change in mobility state of the user, between the first window and the second window. The system 100 identifies the transition state of the user as 'transition state 1 (also referred to as 'first transition state')' if the mobility state of the user changes from the first mobility state to the second mobility state, between the first and second windows. The system 100 identifies transition state of the user as 'transition state 2 (also referred to as 'second transition state')' if the mobility state of the user continues to be the first mobility state (in other words, the user continues to be in the first mobility state), between the first and second windows. The system 100 identifies transition state of the user as 'transition state 3 (also referred to as 'third transition state')' if the mobility state of the user changes from the second mobility state to the first mobility state or if the user continues to be in the second mobility state, between the first and second windows. Based on the identified transition state of the user, the system 100 executes one of a first method, a second method, or a third method so as to filter the estimated heart rate data.

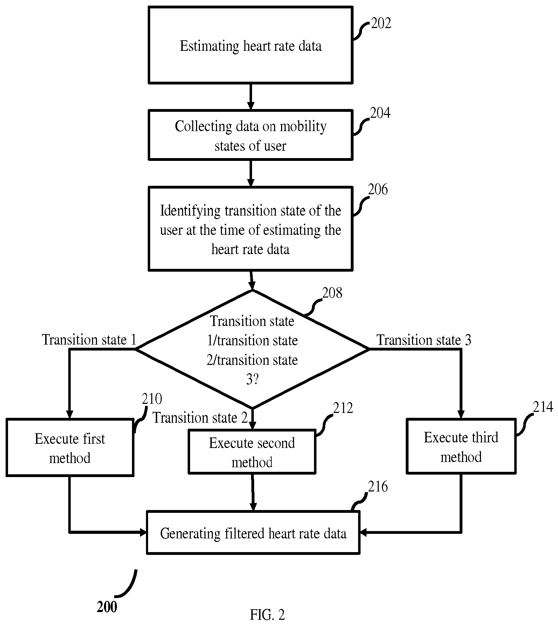

FIG. 2 is a flow diagram depicting the steps involved in the process of heart rate estimation and filtering of estimated heart rate data, in accordance with some embodiments of the present disclosure. The system 100 initially estimates (202) heart rate data of a user being monitored. The system 100 further collects (204) data pertaining to mobility state of the user (mobility state data), at the same time the heart rate measurement is being performed. The system 100 distributes the heart rate data among a plurality of time windows, and then picks heart rate data and corresponding mobility state data from a first window and a second window of the plurality of time windows, at a time, for processing.

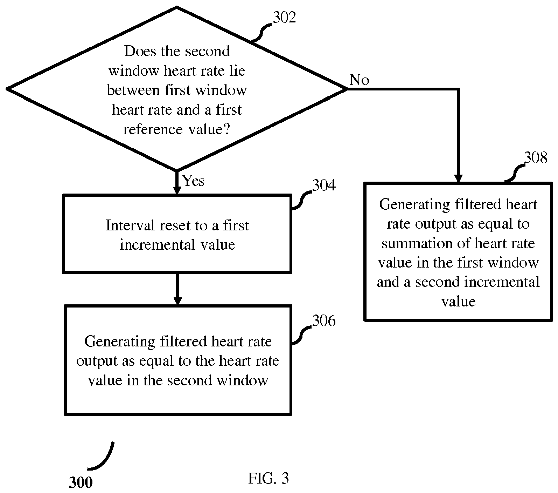

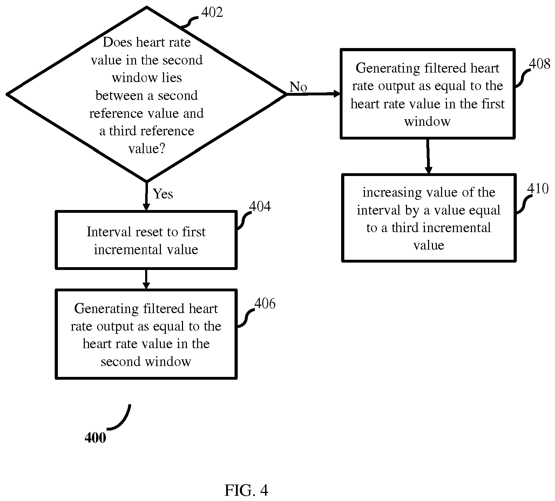

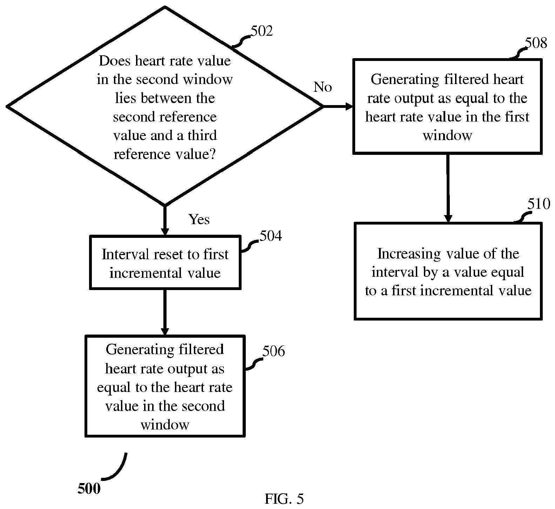

The system 100 identifies (206) transition state of the user, for the two windows being considered, based on the mobility state(s) of the user in the first window and the second window. The transition state of the user is identified as one of a transition state 1, transition state 2, or transition state 3. If the identified transition state is transition state 1, then the system 100 executes (210) a first method (explained in Fig. 3 description). If the identified transition state is transition state 2, then the system 100 executes (212) a second method (explained in Fig. 4 description). If the identified transition state is transition state 3, then the system 100 executes (214) a third method (explained in Fig. 5 description). By executing one of the first method, the second method, and the third method, the system 100 generates (216) a filtered heart rate data.

When the user's mobility state changes, there is a transition from rest to motion or motion to rest. In either case, due to movement, heart rate data of the user may vary. As change in heart rate is considered 'normal' while the mobility state of the user changes, by identifying the transition state and by executing appropriate method (first method or second method or third method), the system 100 applies appropriate correction to the estimated heart rate data. This in turn helps the system 100 to prevent triggering false alarms. In various embodiments, various steps in method 200 may be performed in the same order as depicted in FIG. 2 or in any appropriate alternate order when required. In another embodiment, one or more steps from FIG. 2 maybe skipped.

FIG. 3 is a flow diagram depicting the steps involved in the process of filtering the estimated heart rate data by executing a first method using the system of FIG. 1, in accordance with some embodiments of the present disclosure.

In the first method, the system 100 checks (302) whether heart rate value in the second window lies between heart rate value in a first window and a first reference value. In an embodiment, the first reference value equals summation of heart rate value in the first window and a heart rate change tolerance interval (also referred to as 'interval'). In an embodiment, the value of interval is set to 10. If the heart rate value in the second window lies between the heart rate value in the first window and the first reference value, then the system 100 resets (304) the value of interval to a first incremental value and subsequently generates (306) value of the filtered heart rate data as equal to heart rate data in the second window. In an embodiment, the first reference value equals summation of the interval and heart rate value in the first window. If the heart rate value in the second window does not lie between the heart rate value in the first window and the first reference value, then the system 100 generates (308) value of the filtered heart rate data as equal to summation of heart rate value in the first window and a second incremental value. In various embodiments, values of the interval, the first incremental value, and the second incremental value are decided/selected based on one or more known facts, and may be pre-configured or dynamically configured with the system 100. For example, the first incremental value and the second incremental value may be set to 10. In various embodiments, various steps in method 300 may be performed in the same order as depicted in FIG. 3 or in any appropriate alternate order when required. In another embodiment, one or more steps from FIG. 3 maybe skipped.

FIG. 4 is a flow diagram depicting the steps involved in the process of filtering the estimated heart rate using a second method, in accordance with some embodiments of the present disclosure. While executing the second method, the system 100 checks (402) whether the heart rate value in the second window lies between a second reference value and a third reference value. The second reference value equals summation of heart rate value in the second window and the interval. The third reference value equals difference between the heart rate value in the second window and the interval. If the heart rate value in the second window lies between a second reference value and a third reference value, then the system 100 resets the interval to a value equal to the first incremental value and subsequently generates (406) value of the filtered heart rate data as equal to the heart rate value in the second window. If the heart rate value in the second window does not lie between the second reference value and the third reference value, then the system 100 generates value of the filtered heart rate data (output of the system 100) as equal to the heart rate value in the first window, and subsequently increments the value of the interval by a third incremental value. In various embodiments, values of the first incremental value and the third incremental value may be decided/selected based on known facts, and may be pre-configured dynamically configured with the system 100. In various embodiments, various steps in method 400 may be performed in the same order as depicted in FIG. 4 or in any appropriate alternate order when required. In another embodiment, one or more steps from FIG. 4 maybe skipped.

FIG. 5 is a flow diagram depicting the steps involved in the process of filtering the estimated heart rate using a third method, in accordance with some embodiments of the present disclosure. The system 100 checks (502) whether the heart rate value in the second window lies between the second reference value and the third reference value. If the heart rate value in the second window lies between the second reference value and the third reference value, then the system 100 resets the interval to a value equaling the first incremental value and subsequently generates the value of the filtered heart rate data as equal to the heart rate value in the second window. If the heart rate value in the second window does not lie between the second reference value and the third reference value, then the system 100 generates value of the filtered heart rate data as equal to the heart rate value in the first window, and subsequently increments the value of the interval by a value which is equal to first incremental value. In various embodiments, various steps in method 500 may be performed in the same order as depicted in FIG. 5 or in any appropriate alternate order when required. In another embodiment, one or more steps from FIG. 5 maybe skipped.

The written description describes the subject matter herein to enable any person skilled in the art to make and use the embodiments.

The embodiments of present disclosure herein addresses unresolved problem of heart rate estimation by considering the transition states of the user. The embodiment, thus provides a system and a method which improves the heart rate estimation accuracy utilizing information pertaining to change mobility states of the user at the time the heart rate data is being estimated.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software modules located therein. Thus, the means can include both hardware means and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various modules described herein may be implemented in other modules or combinations of other modules. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, and that the invention be indicated by the following claims.

## Claims

1. A method for heart rate estimation, the method comprising the steps of:
estimating heart rate data of a user by an external system, wherein the estimated heart rate is distributed among a plurality of time windows;
collecting data pertaining to a first mobility state and a second mobility state of the user by the external system, while estimating the heart rate data wherein the first mobility state is a state of rest and the second mobility state is a state of motion; and
processing the estimated heart rate data by a processor (102) and the data pertaining to the first mobility state and the second mobility state,
**characterised in that** the processing comprises:
identifying a transition state of the user as one of a first transition state, a second transition state, or a third transition state, in terms of transition between the first mobility state and the second mobility state while estimating the heart rate data, wherein the first transition state represents transition of the user from the first mobility state to the second mobility state between a first window and a second window of the plurality of time windows, wherein the second transition state represents the user continuing in the first mobility state between the first window and the second window of the plurality of time windows, and wherein the third transition state represents transition of user from the second mobility state to the first mobility state or the user continuing in the second mobility state between the first window and the second window of the plurality of time windows;
filtering the estimated heart rate data by the processor (104) based on the identified transition state of the user, wherein filtering the estimated heart rate data by executing a first method when the identified transition state is the first transition state and wherein filtering the heart rate data by executing a second method if the identified transition state is the second transition state and wherein filtering the heart rate data by executing a third method if the identified transition state is the third transition state,
wherein filtering the heart rate data by executing the first method, the first method, comprises:
checking whether heart rate value in the second window of the plurality of time windows lies between heart rate value in the first window and a first reference value, wherein the first reference value equals summation of heart rate value in the first window and a predefined heart rate change tolerance interval;
if the heart rate value in the second window lies between the heart rate value in the first window and the first reference value, then
resetting value of the tolerance interval equal to a first value; and
generating the filtered heart rate data as equal to heart rate value in the second window as output; and
if the heart rate value in the second window does not lie between the heart rate value in the first window and the first reference value:
generating the filtered heart rate data as equal to summation of heart rate value in the first window and a second value as output.

2. The method as claimed in claim 1, wherein filtering the heart rate data if the identified transition state is the second transition state, by executing the second method, comprises:
checking whether the heart rate value in the second window lies between a second reference value and a third reference value, wherein the second reference value equals summation of heart rate value in the second window and the predefined heart rate change tolerance interval and the third reference value equals difference between the heart rate value in the second window and the predefined heart rate change tolerance interval;
if the heart rate value in the second window lies between a second reference value and a third reference value, then
resetting value of the tolerance interval equal to a first value; and generating the filtered heart rate data as equal to the heart rate value in the second window; and
if the heart rate value in the second window does not lie between the second reference value and the third reference value:
generating the filtered heart rate data as equal to the heart rate value in the first window; and
increasing value of the interval by a value equal to a third value.

3. The method as claimed in claim 1, wherein filtering the heart rate data if the identified transition state is the third transition state, by executing the third method, comprises:
checking whether the heart rate value in the second window lies between a second reference value and a third reference value, wherein the second reference value equals summation of heart rate value in the second window and the predefined heart rate change tolerance interval and the third reference value equals difference between the heart rate value in the second window and the predefined heart rate change tolerance interval;
if the heart rate value in the second window lies between the second reference value and the third reference value, then
resetting value of the tolerance interval equal to a first value; and generating the filtered heart rate data as equal to the heart rate value in the second window; and
if the heart rate value in the second window does not lie between the second reference value and the third reference value:
generating the filtered heart rate data as equal to the heart rate value in the first window; and
increasing value of the interval by the first value.

4. A system (100) for heart rate estimation comprising:
a memory module (102) storing a plurality of instructions;
one or more communication interfaces (110);
an external system; and
one or more hardware processors (104) coupled to the memory module (102) via the one or more communication interfaces (110), wherein the one or more hardware processors are caused by the plurality of instructions to:
estimate heart rate data of a user by the external system, via a heart rate estimation module (106), wherein the estimated heart rate is distributed among a plurality of time windows;
collect data pertaining to a first mobility state and a second mobility state of the user by the external system, while estimating the heart rate data wherein the first mobility state is a state of rest and the second mobility state is a state of motion; and
process the estimated heart rate data and the data pertaining to the first mobility state and the second mobility state, **characterised in that** the processing comprises:
identifying a transition state of the user as one of a first transition state, a second transition state, or a third transition state, in terms of a transition between the first mobility state and the second mobility state while estimating the heart rate data, wherein the first transition state represents transition of the user from the first mobility state to the second mobility state between a first window and a second window of the plurality of time windows, wherein the second transition state represents the user continuing in the first mobility state between the first window and the second window of the plurality of time windows, and wherein the third transition state represents transition of user from the second mobility state to the first mobility state or the user continuing in the second mobility state between the first window and the second window of the plurality of time windows;
filtering the estimated heart rate data based on the identified transition state of the user wherein filtering the estimated heart rate data by executing a first method when the identified transition state is the first transition state and wherein filtering the heart rate data by executing a second method if the identified transition state is the second transition state, and wherein filtering the heart rate data by executing a third method if the identified transition state is the third transition state, via a post processing module (108);
wherein the system filters the heart rate data by executing the first method, if the identified transition state is the first transition state, by:
checking whether heart rate value in a second window of the plurality of time windows lies between heart rate value in a first window of the plurality of time windows and a first reference value, wherein the first reference value equals summation of heart rate value in the first window and a predefined heart rate change tolerance interval;
if the heart rate value in the second window lies between the heart rate value in the first window and the first reference value, then
resetting value of the tolerance interval equal to a first value; and
generating the filtered heart rate data as equal to heart rate value in the second window as output; and
if the heart rate value in the second window does not lie between the heart rate value in the first window and the first reference value:
generating the filtered heart rate data as equal to summation of heart rate value in the first window and second value.

5. The system as claimed in claim 4 wherein the system filters the heart rate data by executing the second method, if the identified transition state is the second transition state, by:
checking whether the heart rate value in the second window lies between a second reference value and a third reference value, wherein the second reference value equals summation of heart rate value in the second window and the predefined heart rate change tolerance interval and the third reference value equals difference between the heart rate value in the second window and the predefined heart rate change tolerance interval;
if the heart rate value in the second window lies between a second reference value and a third reference value, then
resetting value of the tolerance interval equal to a first value; and generating the filtered heart rate data as equal to the heart rate value in the second window;
if the heart rate value in the second window does not lie between a second reference value and a third reference value:
generating the filtered heart rate data as equal to the heart rate value in the first window; and
increasing value of the interval by a value equal to a third value.

6. The system as claimed in claim 4, wherein the system filters the heart rate data by executing third method, if the identified transition state is the third transition state, by:
checking whether the heart rate value in the second window lies between the second reference value and the third reference value, wherein the second reference value equals summation of heart rate value in the second window and
the predefined heart rate change tolerance interval and the third reference value equals difference between the heart rate value in the second window and the predefined heart rate change tolerance interval;
if the heart rate value in the second window lies between the second reference value and the third reference value, then
resetting value of the tolerance interval equal to a first value; and generating the filtered heart rate data as equal to the heart rate value in the second window; and
if the heart rate value in the second window does not lie between the second reference value and the third reference value:
generating the filtered heart rate data as equal to the heart rate value in the first window; and
increasing value of the interval by a first value.

7. One or more non-transitory machine readable information storage mediums comprising one or more instructions which when executed by one or more hardware processors cause:
estimating heart rate data of a user by an external system, wherein the estimated heart rate is distributed among a plurality of time windows;
collecting data pertaining to a first mobility state and a second mobility state of the user by the external system, while estimating the heart rate data wherein the first mobility state is a state of rest and the second mobility state is a state of motion; and
processing the estimated heart rate data and the data pertaining to the first mobility state and the second mobility state, **characterised in that** the processing comprises:
identifying a transition state of the user as one of a first transition state, a second transition state, or a third transition state, in terms of transition between the first mobility state and the second mobility state while estimating the heart rate data, wherein the first transition state represents transition of the user from the first mobility state to the second mobility state between a first window and a second window of the plurality of time windows, wherein the second transition state represents the user continuing in the first mobility state between the first window and the second window of the plurality of time windows, and wherein the third transition state represents transition of user from the second mobility state to the first mobility state or the user continuing in the second mobility state between the first window and the second window of the plurality of time windows;
filtering the estimated heart rate data based on the identified transition state of the user wherein filtering the estimated heart rate data by executing a first method when the identified transition state is the first transition state and wherein filtering the heart rate data by executing a second method if the identified transition state is the second transition state and wherein filtering the heart rate data by executing a third method if the identified transition state is the third transition state,
wherein filtering the estimated heart rate data if the identified transition state is the first transition state, by executing a first method, comprises:
checking whether heart rate value in the second window of the plurality of time windows lies between heart rate value in the first window and a first reference value, wherein the first reference value equals summation of heart rate value in the first window and a predefined heart rate change tolerance interval;
if the heart rate value in the second window lies between the heart rate value in the first window and the first reference value, then
resetting value of the tolerance interval equal to a first value.; and generating the filtered heart rate data as equal to heart rate value in the second window as output; and
if the heart rate value in the second window does not lie between the heart rate value in the first window and the first reference value:
generating the filtered heart rate data as equal to summation of heart rate value in the first window and a second value.

8. The one or more non-transitory machine readable information storage mediums of claim 7, wherein filtering the heart rate data if the identified transition state is the second transition state, by executing the second method, comprises:
checking whether the heart rate value in the second window lies between a second reference value and a third reference value, wherein the second reference value equals summation of heart rate value in the second window and the predefined heart rate change tolerance interval and the third reference value equals difference between the heart rate value in the second window and the predefined heart rate change tolerance interval;
if the heart rate value in the second window lies between a second reference value and a third reference value, then
resetting value of the tolerance interval equal to a first value; and generating the filtered heart rate data as equal to the heart rate value in the second window; and
if the heart rate value in the second window does not lie between the second reference value and the third reference value:
generating the filtered heart rate data as equal to the heart rate value in the first window; and
increasing value of the interval by a value equal to a third value.

9. The one or more non-transitory machine readable information storage mediums of claim 7, wherein filtering the heart rate data if the identified transition state is the third transition state, by executing the third method, comprises:
checking whether the heart rate value in the second window lies between a second reference value and a third reference value, wherein the second reference value equals summation of heart rate value in the second window and the predefined heart rate change tolerance interval and the third reference value equals difference between the heart rate value in the second window and the predefined heart rate change tolerance interval;
if the heart rate value in the second window lies between the second reference value and the third reference value, then
resetting value of the tolerance interval equal to a first value; and generating the filtered heart rate data as equal to the heart rate value in the second window; and
if the heart rate value in the second window does not lie between the second reference value and the third reference value:
generating the filtered heart rate data as equal to the heart rate value in the first window; and
increasing value of the interval by the first value.

## Patentansprüche

1. Verfahren zur Herzfrequenzschätzung, wobei das Verfahren die folgenden Schritte umfasst:
Schätzen von Herzfrequenzdaten eines Benutzers durch ein externes System, wobei die geschätzte Herzfrequenz auf eine Mehrzahl von Zeitfenstern verteilt ist;
Sammeln von Daten, die einen ersten Mobilitätszustand und einen zweiten Mobilitätszustand des Benutzers betreffen, durch das externe System, während die Herzfrequenzdaten geschätzt werden, wobei der erste Mobilitätszustand ein Ruhezustand ist und der zweite Mobilitätszustand ein Bewegungszustand ist; und
Verarbeiten der geschätzten Herzfrequenzdaten durch einen Prozessor (102) und der Daten, die den ersten Mobilitätszustand und den zweiten Mobilitätszustand betreffen, **dadurch gekennzeichnet, dass** die Verarbeitung umfasst:
Identifizieren eines Übergangszustands des Benutzers als einen von einem ersten Übergangszustand, einem zweiten Übergangszustand oder einem dritten Übergangszustand in Bezug auf einen Übergang zwischen dem ersten Mobilitätszustand und dem zweiten Mobilitätszustand, während die Herzfrequenzdaten geschätzt werden, wobei der erste Übergangszustand einen Übergang des Benutzers von dem ersten Mobilitätszustand zu dem zweiten Mobilitätszustand zwischen einem ersten Fenster und einem zweiten Fenster der Mehrzahl von Zeitfenstern darstellt, wobei der zweite Übergangszustand darstellt, dass der Benutzer in dem ersten Mobilitätszustand zwischen dem ersten Fenster und dem zweiten Fenster der Mehrzahl von Zeitfenstern fortfährt, und wobei der dritte Übergangszustand einen Übergang des Benutzers von dem zweiten Mobilitätszustand zu dem ersten Mobilitätszustand darstellt oder dass der Benutzer in dem zweiten Mobilitätszustand zwischen dem ersten Fenster und dem zweiten Fenster der Mehrzahl von Zeitfenstern fortfährt;
Filtern der geschätzten Herzfrequenzdaten durch den Prozessor (104) basierend auf dem identifizierten Übergangszustand des Benutzers, wobei das Filtern der geschätzten Herzfrequenzdaten durch Ausführen eines ersten Verfahrens erfolgt, wenn der identifizierte Übergangszustand der erste Übergangszustand ist, und wobei das Filtern der Herzfrequenzdaten durch Ausführen eines zweiten Verfahrens erfolgt, wenn der identifizierte Übergangszustand der zweite Übergangszustand ist, und wobei das Filtern der Herzfrequenzdaten durch Ausführen eines dritten Verfahrens erfolgt, wenn der identifizierte Übergangszustand der dritte Übergangszustand ist,
wobei das Filtern der Herzfrequenzdaten durch Ausführen des ersten Verfahrens, das erste Verfahren, Folgendes umfasst:
Prüfen, ob ein Herzfrequenzwert in dem zweiten Fenster der Mehrzahl von Zeitfenstern zwischen einem Herzfrequenzwert in dem ersten Fenster und einem ersten Referenzwert liegt, wobei der erste Referenzwert gleich einer Summierung des Herzfrequenzwerts in dem ersten Fenster und einem vordefinierten Herzfrequenzänderungs-Toleranzintervall ist;
wenn der Herzfrequenzwert in dem zweiten Fenster zwischen dem Herzfrequenzwert in dem ersten Fenster und dem ersten Referenzwert liegt, dann
Zurücksetzen des Werts des Toleranzintervalls gleich einem ersten Wert; und
Erzeugen der gefilterten Herzfrequenzdaten als gleich dem Herzfrequenzwert in dem zweiten Fenster als Ausgabe; und
wenn der Herzfrequenzwert in dem zweiten Fenster nicht zwischen dem Herzfrequenzwert in dem ersten Fenster und dem ersten Referenzwert liegt:
Erzeugen der gefilterten Herzfrequenzdaten als gleich einer Summierung des Herzfrequenzwerts in dem ersten Fenster und einem zweiten Wert als Ausgabe.

2. Verfahren nach Anspruch 1, wobei das Filtern der Herzfrequenzdaten, wenn der identifizierte Übergangszustand der zweite Übergangszustand ist, durch Ausführen des zweiten Verfahrens umfasst:
Prüfen, ob der Herzfrequenzwert in dem zweiten Fenster zwischen einem zweiten Referenzwert und einem dritten Referenzwert liegt, wobei der zweite Referenzwert gleich einer Summierung des Herzfrequenzwerts in dem zweiten Fenster und dem vordefinierten Herzfrequenzänderungs-Toleranzintervall ist und der dritte Referenzwert gleich einer Differenz zwischen dem Herzfrequenzwert in dem zweiten Fenster und dem vordefinierten Herzfrequenzänderungs-Toleranzintervall ist;
wenn der Herzfrequenzwert in dem zweiten Fenster zwischen einem zweiten Referenzwert und einem dritten Referenzwert liegt, dann
Zurücksetzen des Werts des Toleranzintervalls gleich einem ersten Wert; und
Erzeugen der gefilterten Herzfrequenzdaten als gleich dem Herzfrequenzwert in dem zweiten Fenster; und
wenn der Herzfrequenzwert in dem zweiten Fenster nicht zwischen dem zweiten Referenzwert und dem dritten Referenzwert liegt:
Erzeugen der gefilterten Herzfrequenzdaten als gleich dem Herzfrequenzwert in dem ersten Fenster; und
Erhöhen des Werts des Intervalls um einen Wert gleich einem dritten Wert.

3. Verfahren nach Anspruch 1, wobei das Filtern der Herzfrequenzdaten, wenn der identifizierte Übergangszustand der dritte Übergangszustand ist, durch Ausführen des dritten Verfahrens umfasst:
Prüfen, ob der Herzfrequenzwert in dem zweiten Fenster zwischen einem zweiten Referenzwert und einem dritten Referenzwert liegt, wobei der zweite Referenzwert gleich einer Summierung des Herzfrequenzwerts in dem zweiten Fenster und dem vordefinierten Herzfrequenzänderungs-Toleranzintervall ist und der dritte Referenzwert gleich einer Differenz zwischen dem Herzfrequenzwert in dem zweiten Fenster und dem vordefinierten Herzfrequenzänderungs-Toleranzintervall ist;
wenn der Herzfrequenzwert in dem zweiten Fenster zwischen dem zweiten Referenzwert und dem dritten Referenzwert liegt, dann
Zurücksetzen des Werts des Toleranzintervalls gleich einem ersten Wert; und
Erzeugen der gefilterten Herzfrequenzdaten als gleich dem Herzfrequenzwert in dem zweiten Fenster; und
wenn der Herzfrequenzwert in dem zweiten Fenster nicht zwischen dem zweiten Referenzwert und dem dritten Referenzwert liegt:
Erzeugen der gefilterten Herzfrequenzdaten als gleich dem Herzfrequenzwert in dem ersten Fenster; und
Erhöhen des Werts des Intervalls um den ersten Wert.

4. System (100) zur Herzfrequenzschätzung, umfassend:
ein Speichermodul (102), das eine Mehrzahl von Anweisungen speichert;
eine oder mehrere Kommunikationsschnittstellen (110);
ein externes System; und
einen oder mehrere Hardwareprozessoren (104), die mit dem Speichermodul (102) über die eine oder die mehreren Kommunikationsschnittstellen (110) gekoppelt sind, wobei der eine oder die mehreren Hardwareprozessoren durch die Mehrzahl von Anweisungen veranlasst werden zum:
Schätzen von Herzfrequenzdaten eines Benutzers durch das externe System über ein Herzfrequenzschätzmodul (106), wobei die geschätzte Herzfrequenz auf eine Mehrzahl von Zeitfenstern verteilt ist;
Sammeln von Daten, die einen ersten Mobilitätszustand und einen zweiten Mobilitätszustand des Benutzers betreffen, durch das externe System, während die Herzfrequenzdaten geschätzt werden, wobei der erste Mobilitätszustand ein Ruhezustand ist und der zweite Mobilitätszustand ein Bewegungszustand ist; und
Verarbeiten der geschätzten Herzfrequenzdaten und der Daten, die den ersten Mobilitätszustand und den zweiten Mobilitätszustand betreffen, **dadurch gekennzeichnet, dass** die Verarbeitung umfasst:
Identifizieren eines Übergangszustands des Benutzers als einen von einem ersten Übergangszustand, einem zweiten Übergangszustand oder einem dritten Übergangszustand in Bezug auf einen Übergang zwischen dem ersten Mobilitätszustand und dem zweiten Mobilitätszustand, während die Herzfrequenzdaten geschätzt werden, wobei der erste Übergangszustand einen Übergang des Benutzers von dem ersten Mobilitätszustand zu dem zweiten Mobilitätszustand zwischen einem ersten Fenster und einem zweiten Fenster der Mehrzahl von Zeitfenstern darstellt, wobei der zweite Übergangszustand darstellt, dass der Benutzer in dem ersten Mobilitätszustand zwischen dem ersten Fenster und dem zweiten Fenster der Mehrzahl von Zeitfenstern fortfährt, und wobei der dritte Übergangszustand einen Übergang des Benutzers von dem zweiten Mobilitätszustand zu dem ersten Mobilitätszustand darstellt oder dass der Benutzer in dem zweiten Mobilitätszustand zwischen dem ersten Fenster und dem zweiten Fenster der Mehrzahl von Zeitfenstern fortfährt;
Filtern der geschätzten Herzfrequenzdaten basierend auf dem identifizierten Übergangszustand des Benutzers, wobei das Filtern der geschätzten Herzfrequenzdaten durch Ausführen eines ersten Verfahrens erfolgt, wenn der identifizierte Übergangszustand der erste Übergangszustand ist, und wobei das Filtern der Herzfrequenzdaten durch Ausführen eines zweiten Verfahrens erfolgt, wenn der identifizierte Übergangszustand der zweite Übergangszustand ist, und wobei das Filtern der Herzfrequenzdaten durch Ausführen eines dritten Verfahrens erfolgt, wenn der identifizierte Übergangszustand der dritte Übergangszustand ist, über ein Nachverarbeitungsmodul (108);
wobei das System die Herzfrequenzdaten durch Ausführen des ersten Verfahrens filtert, wenn der identifizierte Übergangszustand der erste Übergangszustand ist, durch:
Prüfen, ob ein Herzfrequenzwert in einem zweiten Fenster der Mehrzahl von Zeitfenstern zwischen einem Herzfrequenzwert in einem ersten Fenster der Mehrzahl von Zeitfenstern und einem ersten Referenzwert liegt, wobei der erste Referenzwert gleich einer Summierung des Herzfrequenzwerts in dem ersten Fenster und einem vordefinierten Herzfrequenzänderungs-Toleranzintervall ist;
wenn der Herzfrequenzwert in dem zweiten Fenster zwischen dem Herzfrequenzwert in dem ersten Fenster und dem ersten Referenzwert liegt, dann
Zurücksetzen des Werts des Toleranzintervalls gleich einem ersten Wert; und
Erzeugen der gefilterten Herzfrequenzdaten als gleich dem Herzfrequenzwert in dem zweiten Fenster als Ausgabe; und
wenn der Herzfrequenzwert in dem zweiten Fenster nicht zwischen dem Herzfrequenzwert in dem ersten Fenster und dem ersten Referenzwert liegt:
Erzeugen der gefilterten Herzfrequenzdaten als gleich einer Summierung des Herzfrequenzwerts in dem ersten Fenster und einem zweiten Wert.

5. System nach Anspruch 4, wobei das System die Herzfrequenzdaten durch Ausführen des zweiten Verfahrens filtert, wenn der identifizierte Übergangszustand der zweite Übergangszustand ist, durch:
Prüfen, ob der Herzfrequenzwert in dem zweiten Fenster zwischen einem zweiten Referenzwert und einem dritten Referenzwert liegt, wobei der zweite Referenzwert gleich einer Summierung des Herzfrequenzwerts in dem zweiten Fenster und dem vordefinierten Herzfrequenzänderungs-Toleranzintervall ist und der dritte Referenzwert gleich einer Differenz zwischen dem Herzfrequenzwert in dem zweiten Fenster und dem vordefinierten Herzfrequenzänderungs-Toleranzintervall ist;
wenn der Herzfrequenzwert in dem zweiten Fenster zwischen einem zweiten Referenzwert und einem dritten Referenzwert liegt, dann
Zurücksetzen des Werts des Toleranzintervalls gleich einem ersten Wert; und
Erzeugen der gefilterten Herzfrequenzdaten als gleich dem Herzfrequenzwert in dem zweiten Fenster;
wenn der Herzfrequenzwert in dem zweiten Fenster nicht zwischen einem zweiten Referenzwert und einem dritten Referenzwert liegt:
Erzeugen der gefilterten Herzfrequenzdaten als gleich dem Herzfrequenzwert in dem ersten Fenster; und
Erhöhen des Werts des Intervalls um einen Wert gleich einem dritten Wert.

6. System nach Anspruch 4, wobei das System die Herzfrequenzdaten durch Ausführen des dritten Verfahrens filtert, wenn der identifizierte Übergangszustand der dritte Übergangszustand ist, durch:
Prüfen, ob der Herzfrequenzwert in dem zweiten Fenster zwischen dem zweiten Referenzwert und dem dritten Referenzwert liegt, wobei der zweite Referenzwert gleich einer Summierung des Herzfrequenzwerts in dem zweiten Fenster und dem vordefinierten Herzfrequenzänderungs-Toleranzintervall ist und der dritte Referenzwert gleich einer Differenz zwischen dem Herzfrequenzwert in dem zweiten Fenster und dem vordefinierten Herzfrequenzänderungs-Toleranzintervall ist;
wenn der Herzfrequenzwert in dem zweiten Fenster zwischen dem zweiten Referenzwert und dem dritten Referenzwert liegt, dann
Zurücksetzen des Werts des Toleranzintervalls gleich einem ersten Wert; und
Erzeugen der gefilterten Herzfrequenzdaten als gleich dem Herzfrequenzwert in dem zweiten Fenster; und
wenn der Herzfrequenzwert in dem zweiten Fenster nicht zwischen dem zweiten Referenzwert und dem dritten Referenzwert liegt:
Erzeugen der gefilterten Herzfrequenzdaten als gleich dem Herzfrequenzwert in dem ersten Fenster; und
Erhöhen des Werts des Intervalls um einen ersten Wert.

7. Ein oder mehrere nichtflüchtige maschinenlesbare Informationsspeichermedien, die eine oder mehrere Anweisungen umfassen, die, wenn sie durch einen oder mehrere Hardwareprozessoren ausgeführt werden, veranlassen zum:
Schätzen von Herzfrequenzdaten eines Benutzers durch ein externes System, wobei die geschätzte Herzfrequenz auf eine Mehrzahl von Zeitfenstern verteilt ist;
Sammeln von Daten, die einen ersten Mobilitätszustand und einen zweiten Mobilitätszustand des Benutzers betreffen, durch das externe System, während die Herzfrequenzdaten geschätzt werden, wobei der erste Mobilitätszustand ein Ruhezustand ist und der zweite Mobilitätszustand ein Bewegungszustand ist; und
Verarbeiten der geschätzten Herzfrequenzdaten und der Daten, die den ersten Mobilitätszustand und den zweiten Mobilitätszustand betreffen, **dadurch gekennzeichnet, dass** die Verarbeitung umfasst:
Identifizieren eines Übergangszustands des Benutzers als einen von einem ersten Übergangszustand, einem zweiten Übergangszustand oder einem dritten Übergangszustand in Bezug auf einen Übergang zwischen dem ersten Mobilitätszustand und dem zweiten Mobilitätszustand, während die Herzfrequenzdaten geschätzt werden, wobei der erste Übergangszustand einen Übergang des Benutzers von dem ersten Mobilitätszustand zu dem zweiten Mobilitätszustand zwischen einem ersten Fenster und einem zweiten Fenster der Mehrzahl von Zeitfenstern darstellt, wobei der zweite Übergangszustand darstellt, dass der Benutzer in dem ersten Mobilitätszustand zwischen dem ersten Fenster und dem zweiten Fenster der Mehrzahl von Zeitfenstern fortfährt, und wobei der dritte Übergangszustand einen Übergang des Benutzers von dem zweiten Mobilitätszustand zu dem ersten Mobilitätszustand darstellt oder dass der Benutzer in dem zweiten Mobilitätszustand zwischen dem ersten Fenster und dem zweiten Fenster der Mehrzahl von Zeitfenstern fortfährt;
Filtern der geschätzten Herzfrequenzdaten basierend auf dem identifizierten Übergangszustand des Benutzers, wobei das Filtern der geschätzten Herzfrequenzdaten durch Ausführen eines ersten Verfahrens erfolgt, wenn der identifizierte Übergangszustand der erste Übergangszustand ist, und wobei das Filtern der Herzfrequenzdaten durch Ausführen eines zweiten Verfahrens erfolgt, wenn der identifizierte Übergangszustand der zweite Übergangszustand ist, und wobei das Filtern der Herzfrequenzdaten durch Ausführen eines dritten Verfahrens erfolgt, wenn der identifizierte Übergangszustand der dritte Übergangszustand ist,
wobei das Filtern der geschätzten Herzfrequenzdaten, wenn der identifizierte Übergangszustand der erste Übergangszustand ist, durch Ausführen eines ersten Verfahrens umfasst:
Prüfen, ob ein Herzfrequenzwert in dem zweiten Fenster der Mehrzahl von Zeitfenstern zwischen einem Herzfrequenzwert in dem ersten Fenster und einem ersten Referenzwert liegt, wobei der erste Referenzwert gleich einer Summierung des Herzfrequenzwerts in dem ersten Fenster und einem vordefinierten Herzfrequenzänderungs-Toleranzintervall ist;
wenn der Herzfrequenzwert in dem zweiten Fenster zwischen dem Herzfrequenzwert in dem ersten Fenster und dem ersten Referenzwert liegt, dann
Zurücksetzen des Werts des Toleranzintervalls gleich einem ersten Wert; und
Erzeugen der gefilterten Herzfrequenzdaten als gleich dem Herzfrequenzwert in dem zweiten Fenster als Ausgabe; und wenn der Herzfrequenzwert in dem zweiten Fenster nicht zwischen dem Herzfrequenzwert in dem ersten Fenster und dem ersten Referenzwert liegt:
Erzeugen der gefilterten Herzfrequenzdaten als gleich einer Summierung des Herzfrequenzwerts in dem ersten Fenster und einem zweiten Wert.

8. Ein oder mehrere nichtflüchtige maschinenlesbare Informationsspeichermedien nach Anspruch 7, wobei das Filtern der Herzfrequenzdaten, wenn der identifizierte Übergangszustand der zweite Übergangszustand ist, durch Ausführen des zweiten Verfahrens umfasst:
Prüfen, ob der Herzfrequenzwert in dem zweiten Fenster zwischen einem zweiten Referenzwert und einem dritten Referenzwert liegt, wobei der zweite Referenzwert gleich einer Summierung des Herzfrequenzwerts in dem zweiten Fenster und dem vordefinierten Herzfrequenzänderungs-Toleranzintervall ist und der dritte Referenzwert gleich einer Differenz zwischen dem Herzfrequenzwert in dem zweiten Fenster und dem vordefinierten Herzfrequenzänderungs-Toleranzintervall ist;
wenn der Herzfrequenzwert in dem zweiten Fenster zwischen einem zweiten Referenzwert und einem dritten Referenzwert liegt, dann
Zurücksetzen des Werts des Toleranzintervalls gleich einem ersten Wert; und
Erzeugen der gefilterten Herzfrequenzdaten als gleich dem Herzfrequenzwert in dem zweiten Fenster; und
wenn der Herzfrequenzwert in dem zweiten Fenster nicht zwischen dem zweiten Referenzwert und dem dritten Referenzwert liegt:
Erzeugen der gefilterten Herzfrequenzdaten als gleich dem Herzfrequenzwert in dem ersten Fenster; und
Erhöhen des Werts des Intervalls um einen Wert gleich einem dritten Wert.

9. Ein oder mehrere nichtflüchtige maschinenlesbare Informationsspeichermedien nach Anspruch 7, wobei das Filtern der Herzfrequenzdaten, wenn der identifizierte Übergangszustand der dritte Übergangszustand ist, durch Ausführen des dritten Verfahrens umfasst:
Prüfen, ob der Herzfrequenzwert in dem zweiten Fenster zwischen einem zweiten Referenzwert und einem dritten Referenzwert liegt, wobei der zweite Referenzwert gleich einer Summierung des Herzfrequenzwerts in dem zweiten Fenster und dem vordefinierten Herzfrequenzänderungs-Toleranzintervall ist und der dritte Referenzwert gleich einer Differenz zwischen dem Herzfrequenzwert in dem zweiten Fenster und dem vordefinierten Herzfrequenzänderungs-Toleranzintervall ist;
wenn der Herzfrequenzwert in dem zweiten Fenster zwischen dem zweiten Referenzwert und dem dritten Wert liegt, dann
Zurücksetzen des Werts des Toleranzintervalls gleich einem ersten Wert; und
Erzeugen der gefilterten Herzfrequenzdaten als gleich dem Herzfrequenzwert in dem zweiten Fenster; und
wenn der Herzfrequenzwert in dem zweiten Fenster nicht zwischen dem zweiten Referenzwert und dem dritten Referenzwert liegt:
Erzeugen der gefilterten Herzfrequenzdaten als gleich dem Herzfrequenzwert in dem ersten Fenster; und
Erhöhen des Werts des Intervalls um den ersten Wert.

## Revendications

1. Procédé d'estimation de fréquence cardiaque, le procédé comprenant les étapes ci-dessous consistant à :
estimer des données de fréquence cardiaque d'un utilisateur par le biais d'un système externe, dans lequel la fréquence cardiaque estimée est répartie parmi une pluralité de fenêtres temporelles ;
recueillir des données se rapportant à un premier état de mobilité et à un deuxième état de mobilité de l'utilisateur, par le biais du système externe, tout en estimant les données de fréquence cardiaque, dans lequel le premier état de mobilité est un état de repos et le deuxième état de mobilité est un état de mouvement ; et
traiter les données de fréquence cardiaque estimées, par le biais d'un processeur (102), et les données se rapportant au premier état de mobilité et au deuxième état de mobilité, **caractérisé en ce que** l'étape de traitement comprend les étapes ci-dessous consistant à :
identifier un état de transition de l'utilisateur en tant que l'un parmi un premier état de transition, un deuxième état de transition ou un troisième état de transition, en termes de transition entre le premier état de mobilité et le deuxième état de mobilité, tout en estimant les données de fréquence cardiaque, dans lequel le premier état de transition représente une transition, de l'utilisateur, du premier état de mobilité au deuxième état de mobilité entre une première fenêtre et une deuxième fenêtre de la pluralité de fenêtres temporelle, dans lequel le deuxième état de transition représente la poursuite, par l'utilisateur, du premier état de mobilité entre la première fenêtre et la deuxième fenêtre de la pluralité de fenêtres temporelles, et dans lequel le troisième état de transition représente une transition, de l'utilisateur, du deuxième état de mobilité au premier état de mobilité ou la poursuite, par l'utilisateur, du deuxième état de mobilité entre la première fenêtre et la deuxième fenêtre de la pluralité de fenêtres temporelles ;
filtrer les données de fréquence cardiaque estimées, par le biais du processeur (104), sur la base de l'état de transition identifié de l'utilisateur, dans laquelle l'étape de filtrage des données de fréquence cardiaque estimées est mise en oeuvre en exécutant un premier procédé lorsque l'état de transition identifié est le premier état de transition, l'étape de filtrage des données de fréquence cardiaque est mise en oeuvre en exécutant un deuxième procédé si l'état de transition identifié est le deuxième état de transition et l'étape de filtrage des données de fréquence cardiaque est mise en oeuvre en exécutant un troisième procédé si l'état de transition identifié est le troisième état de transition ;
dans laquelle l'étape de filtrage des données de fréquence cardiaque mise en oeuvre en exécutant le premier procédé comprend les étapes ci-dessous consistant à :
vérifier si une valeur de fréquence cardiaque dans la deuxième fenêtre de la pluralité de fenêtres temporelles se situe entre une valeur de fréquence cardiaque dans la première fenêtre et une première valeur de référence, dans laquelle la première valeur de référence est égale à la somme de la valeur de fréquence cardiaque dans la première fenêtre et d'un intervalle de tolérance de changement de fréquence cardiaque prédéfini ;
si la valeur de fréquence cardiaque dans la deuxième fenêtre se situe entre la valeur de fréquence cardiaque dans la première fenêtre et la première valeur de référence, alors
réinitialiser la valeur de l'intervalle de tolérance afin qu'elle soit égale à une première valeur ; et
générer en sortie les données de fréquence cardiaque filtrées comme étant égales à la valeur de fréquence cardiaque dans la deuxième fenêtre ; et
si la valeur de fréquence cardiaque dans la deuxième fenêtre ne se situe pas entre la valeur de fréquence cardiaque dans la première fenêtre et la première valeur de référence :
générer en sortie les données de fréquence cardiaque filtrées comme étant égales à la somme de la valeur de fréquence cardiaque dans la première fenêtre et d'une deuxième valeur.

2. Procédé selon la revendication 1, dans lequel l'étape de filtrage des données de fréquence cardiaque si l'état de transition identifié est le deuxième état de transition, mise en oeuvre en exécutant le deuxième procédé, comprend les étapes ci-dessous consistant à :
vérifier si la valeur de fréquence cardiaque dans la deuxième fenêtre se situe entre une deuxième valeur de référence et une troisième valeur de référence, dans lequel la deuxième valeur de référence est égale à la somme d'une valeur de fréquence cardiaque dans la deuxième fenêtre et de l'intervalle de tolérance de changement de fréquence cardiaque prédéfini, et la troisième valeur de référence est égale à la différence entre la valeur de fréquence cardiaque dans la deuxième fenêtre et l'intervalle de tolérance de changement de fréquence cardiaque prédéfini ;
si la valeur de fréquence cardiaque dans la deuxième fenêtre se situe entre une deuxième valeur de référence et une troisième valeur de référence, alors
réinitialiser la valeur de l'intervalle de tolérance afin qu'elle soit égale à une première valeur ; et
générer les données de fréquence cardiaque filtrées comme étant égales à la valeur de fréquence cardiaque dans la deuxième fenêtre ; et
si la valeur de fréquence cardiaque dans la deuxième fenêtre ne se situe pas entre la deuxième valeur de référence et la troisième valeur de référence :
générer les données de fréquence cardiaque filtrées comme étant égales à la valeur de fréquence cardiaque dans la première fenêtre ; et
augmenter la valeur de l'intervalle, d'une valeur égale à une troisième valeur.

3. Procédé selon la revendication 1, dans lequel l'étape de filtrage des données de fréquence cardiaque si l'état de transition identifié est le troisième état de transition, en exécutant le troisième procédé, comprend les étapes ci-dessous consistant à :
vérifier si la valeur de fréquence cardiaque dans la deuxième fenêtre se situe entre une deuxième valeur de référence et une troisième valeur de référence, dans laquelle la deuxième valeur de référence est égale à la somme d'une valeur de fréquence cardiaque dans la deuxième fenêtre et de l'intervalle de tolérance de changement de fréquence cardiaque prédéfini, et la troisième valeur de référence est égale à la différence entre la valeur de fréquence cardiaque dans la deuxième fenêtre et l'intervalle de tolérance de changement de fréquence cardiaque prédéfini ;
si la valeur de fréquence cardiaque dans la deuxième fenêtre se situe entre la deuxième valeur de référence et la troisième valeur de référence, alors
réinitialiser la valeur de l'intervalle de tolérance afin qu'elle soit égale à une première valeur ; et
générer les données de fréquence cardiaque filtrées comme étant égales à la valeur de fréquence cardiaque dans la deuxième fenêtre ; et
si la valeur de fréquence cardiaque dans la deuxième fenêtre ne se situe pas entre la deuxième valeur de référence et la troisième valeur de référence :
générer les données de fréquence cardiaque filtrées comme étant égales à la valeur de fréquence cardiaque dans la première fenêtre ; et
augmenter la valeur de l'intervalle, de la première valeur.

4. Système (100) d'estimation de fréquence cardiaque, comprenant :
un module de mémoire (102) stockant une pluralité d'instructions ;
une ou plusieurs interfaces de communication (110) ;
un système externe ; et
un ou plusieurs processeurs matériels (104) couplés au module de mémoire (102) par l'intermédiaire de ladite une ou desdites plusieurs interfaces de communication (110), dans lesquels ledit un ou lesdits plusieurs processeurs matériels sont amenés, par la pluralité d'instructions, à mettre en oeuvre les étapes ci-dessous consistant à :
estimer des données de fréquence cardiaque d'un utilisateur par le biais du système externe, par l'intermédiaire d'un module d'estimation de fréquence cardiaque (106), dans lequel la fréquence cardiaque estimée est répartie parmi une pluralité de fenêtres temporelles ;
recueillir des données se rapportant à un premier état de mobilité et à un deuxième état de mobilité de l'utilisateur, par le biais du système externe, tout en estimant les données de fréquence cardiaque, dans lequel le premier état de mobilité est un état de repos et le deuxième état de mobilité est un état de mouvement ; et
traiter les données de fréquence cardiaque estimées et les données se rapportant au premier état de mobilité et au deuxième état de mobilité, **caractérisé en ce que** l'étape de traitement comprend les étapes ci-dessous consistant à :
identifier un état de transition de l'utilisateur en tant que l'un parmi un premier état de transition, un deuxième état de transition ou un troisième état de transition, en termes de transition entre le premier état de mobilité et le deuxième état de mobilité, tout en estimant les données de fréquence cardiaque, dans lequel le premier état de transition représente une transition, de l'utilisateur, du premier état de mobilité au deuxième état de mobilité entre une première fenêtre et une deuxième fenêtre de la pluralité de fenêtres temporelle, dans lequel le deuxième état de transition représente la poursuite, par l'utilisateur, du premier état de mobilité entre la première fenêtre et la deuxième fenêtre de la pluralité de fenêtres temporelles, et dans lequel le troisième état de transition représente une transition, de l'utilisateur, du deuxième état de mobilité au premier état de mobilité ou la poursuite, par l'utilisateur, du deuxième état de mobilité entre la première fenêtre et la deuxième fenêtre de la pluralité de fenêtres temporelles ;
filtrer les données de fréquence cardiaque estimées sur la base de l'état de transition identifié de l'utilisateur, dans laquelle l'étape de filtrage des données de fréquence cardiaque estimées est mise en oeuvre en exécutant un premier procédé lorsque l'état de transition identifié est le premier état de transition, l'étape de filtrage des données de fréquence cardiaque est mise en oeuvre en exécutant un deuxième procédé si l'état de transition identifié est le deuxième état de transition et l'étape de filtrage des données de fréquence cardiaque est mise en oeuvre en exécutant un troisième procédé si l'état de transition identifié est le troisième état de transition, par l'intermédiaire du module de post-traitement (108) ;
dans lequel le système filtre les données de fréquence cardiaque en exécutant le premier procédé, si l'état de transition identifié est le premier état de transition, en mettant en oeuvre les étapes ci-dessous consistant à :
vérifier si une valeur de fréquence cardiaque dans la deuxième fenêtre de la pluralité de fenêtres temporelles se situe entre une valeur de fréquence cardiaque d'une première fenêtre de la pluralité de fenêtres temporelles et une première valeur de référence, dans laquelle la première valeur de référence est égale à la somme de la valeur de fréquence cardiaque dans la première fenêtre et d'un intervalle de tolérance de changement de fréquence cardiaque prédéfini ;
si la valeur de fréquence cardiaque dans la deuxième fenêtre se situe entre la valeur de fréquence cardiaque dans la première fenêtre et la première valeur de référence, alors
réinitialiser la valeur de l'intervalle de tolérance afin qu'elle soit égale à une première valeur ; et
générer en sortie les données de fréquence cardiaque filtrées comme étant égales à la valeur de fréquence cardiaque dans la deuxième fenêtre ; et
si la valeur de fréquence cardiaque dans la deuxième fenêtre ne se situe pas entre la valeur de fréquence cardiaque dans la première fenêtre et la première valeur de référence :
générer les données de fréquence cardiaque filtrées comme étant égales à la somme de la valeur de fréquence cardiaque dans la première fenêtre et d'une deuxième valeur.

5. Système selon la revendication 4, dans lequel le système filtre les données de fréquence cardiaque en exécutant le deuxième procédé, si l'état de transition identifié est le deuxième état de transition, en mettant en oeuvre les étapes ci-dessous consistant à :
vérifier si la valeur de fréquence cardiaque dans la deuxième fenêtre se situe entre une deuxième valeur de référence et une troisième valeur de référence, dans lequel la deuxième valeur de référence est égale à la somme d'une valeur de fréquence cardiaque dans la deuxième fenêtre et de l'intervalle de tolérance de changement de fréquence cardiaque prédéfini, et la troisième valeur de référence est égale à la différence entre la valeur de fréquence cardiaque dans la deuxième fenêtre et l'intervalle de tolérance de changement de fréquence cardiaque prédéfini ;
si la valeur de fréquence cardiaque dans la deuxième fenêtre se situe entre une deuxième valeur de référence et une troisième valeur de référence, alors
réinitialiser la valeur de l'intervalle de tolérance afin qu'elle soit égale à une première valeur ; et
générer les données de fréquence cardiaque filtrées comme étant égales à la valeur de fréquence cardiaque dans la deuxième fenêtre ; et
si la valeur de fréquence cardiaque dans la deuxième fenêtre ne se situe pas entre la deuxième valeur de référence et la troisième valeur de référence :
générer les données de fréquence cardiaque filtrées comme étant égales à la valeur de fréquence cardiaque dans la première fenêtre ; et
augmenter la valeur de l'intervalle, d'une valeur égale à une troisième valeur.

6. Système selon la revendication 4, dans lequel le système filtre les données de fréquence cardiaque en exécutant le troisième procédé, si l'état de transition identifié est le troisième état de transition, en mettant en oeuvre les étapes ci-dessous consistant à :
vérifier si la valeur de fréquence cardiaque dans la deuxième fenêtre se situe entre la deuxième valeur de référence et la troisième valeur de référence, dans laquelle la deuxième valeur de référence est égale à la somme d'une valeur de fréquence cardiaque dans la deuxième fenêtre et de l'intervalle de tolérance de changement de fréquence cardiaque prédéfini, et la troisième valeur de référence est égale à la différence entre la valeur de fréquence cardiaque dans la deuxième fenêtre et l'intervalle de tolérance de changement de fréquence cardiaque prédéfini ;
si la valeur de fréquence cardiaque dans la deuxième fenêtre se situe entre la deuxième valeur de référence et la troisième valeur de référence, alors
réinitialiser la valeur de l'intervalle de tolérance afin qu'elle soit égale à une première valeur ; et
générer les données de fréquence cardiaque filtrées comme étant égales à la valeur de fréquence cardiaque dans la deuxième fenêtre ; et
si la valeur de fréquence cardiaque dans la deuxième fenêtre ne se situe pas entre la deuxième valeur de référence et la troisième valeur de référence :
générer les données de fréquence cardiaque filtrées comme étant égales à la valeur de fréquence cardiaque dans la première fenêtre ; et
augmenter la valeur de l'intervalle, de la première valeur.

7. Un ou plusieurs supports non transitoires de stockage d'informations lisibles par machine comprenant une ou plusieurs instructions qui, lorsqu'elles sont exécutées par un ou plusieurs processeurs matériels, occasionnent la mise en oeuvre des étapes ci-dessous consistant à :
estimer des données de fréquence cardiaque d'un utilisateur par le biais d'un système externe, dans lequel la fréquence cardiaque estimée est répartie parmi une pluralité de fenêtres temporelles ;
recueillir des données se rapportant à un premier état de mobilité et à un deuxième état de mobilité de l'utilisateur, par le biais du système externe, tout en estimant les données de fréquence cardiaque, dans lequel le premier état de mobilité est un état de repos et le deuxième état de mobilité est un état de mouvement ; et
traiter les données de fréquence cardiaque estimées et les données se rapportant au premier état de mobilité et au deuxième état de mobilité, **caractérisé en ce que** l'étape de traitement comprend les étapes ci-dessous consistant à :
identifier un état de transition de l'utilisateur en tant que l'un parmi un premier état de transition, un deuxième état de transition ou un troisième état de transition, en termes de transition entre le premier état de mobilité et le deuxième état de mobilité, tout en estimant les données de fréquence cardiaque, dans lequel le premier état de transition représente une transition, de l'utilisateur, du premier état de mobilité au deuxième état de mobilité entre une première fenêtre et une deuxième fenêtre de la pluralité de fenêtres temporelle, dans lequel le deuxième état de transition représente la poursuite, par l'utilisateur, du premier état de mobilité entre la première fenêtre et la deuxième fenêtre de la pluralité de fenêtres temporelles, et dans lequel le troisième état de transition représente une transition, de l'utilisateur, du deuxième état de mobilité au premier état de mobilité ou la poursuite, par l'utilisateur, du deuxième état de mobilité entre la première fenêtre et la deuxième fenêtre de la pluralité de fenêtres temporelles ;
filtrer les données de fréquence cardiaque estimées sur la base de l'état de transition identifié de l'utilisateur, dans laquelle l'étape de filtrage des données de fréquence cardiaque estimées est mise en oeuvre en exécutant un premier procédé lorsque l'état de transition identifié est le premier état de transition, l'étape de filtrage des données de fréquence cardiaque est mise en oeuvre en exécutant un deuxième procédé si l'état de transition identifié est le deuxième état de transition et l'étape de filtrage des données de fréquence cardiaque est mise en oeuvre en exécutant un troisième procédé si l'état de transition identifié est le troisième état de transition ;
dans laquelle l'étape de filtrage des données de fréquence cardiaque, si l'état de transition identifié est le premier état de transition, en exécutant le premier procédé, comprend les étapes ci-dessous consistant à :
vérifier si une valeur de fréquence cardiaque dans la deuxième fenêtre de la pluralité de fenêtres temporelles se situe entre une valeur de fréquence cardiaque dans la première fenêtre et une première valeur de référence, dans laquelle la première valeur de référence est égale à la somme de la valeur de fréquence cardiaque dans la première fenêtre et d'un intervalle de tolérance de changement de fréquence cardiaque prédéfini ;
si la valeur de fréquence cardiaque dans la deuxième fenêtre se situe entre la valeur de fréquence cardiaque dans la première fenêtre et la première valeur de référence, alors
réinitialiser la valeur de l'intervalle de tolérance afin qu'elle soit égale à une première valeur ; et
générer en sortie les données de fréquence cardiaque filtrées comme étant égales à la valeur de fréquence cardiaque dans la deuxième fenêtre ; et
si la valeur de fréquence cardiaque dans la deuxième fenêtre ne se situe pas entre la valeur de fréquence cardiaque dans la première fenêtre et la première valeur de référence :
générer les données de fréquence cardiaque filtrées comme étant égales à la somme de la valeur de fréquence cardiaque dans la première fenêtre et d'une deuxième valeur.

8. Ledit un ou lesdits plusieurs supports non transitoires de stockage d'informations lisibles par machine selon la revendication 7, dans lesquels l'étape de filtrage des données de fréquence cardiaque, si l'état de transition identifié est le deuxième état de transition, en exécutant le deuxième procédé, comprend les étapes ci-dessous consistant à :
vérifier si la valeur de fréquence cardiaque dans la deuxième fenêtre se situe entre une deuxième valeur de référence et une troisième valeur de référence, dans lequel la deuxième valeur de référence est égale à la somme d'une valeur de fréquence cardiaque dans la deuxième fenêtre et de l'intervalle de tolérance de changement de fréquence cardiaque prédéfini, et la troisième valeur de référence est égale à la différence entre la valeur de fréquence cardiaque dans la deuxième fenêtre et l'intervalle de tolérance de changement de fréquence cardiaque prédéfini ;
si la valeur de fréquence cardiaque dans la deuxième fenêtre se situe entre une deuxième valeur de référence et une troisième valeur de référence, alors
réinitialiser la valeur de l'intervalle de tolérance afin qu'elle soit égale à une première valeur ; et
générer les données de fréquence cardiaque filtrées comme étant égales à la valeur de fréquence cardiaque dans la deuxième fenêtre ; et
si la valeur de fréquence cardiaque dans la deuxième fenêtre ne se situe pas entre la deuxième valeur de référence et la troisième valeur de référence :
générer les données de fréquence cardiaque filtrées comme étant égales à la valeur de fréquence cardiaque dans la première fenêtre ; et
augmenter la valeur de l'intervalle, d'une valeur égale à une troisième valeur.

9. Ledit un ou lesdits plusieurs supports non transitoires de stockage d'informations lisibles par machine selon la revendication 7, dans lesquels l'étape de filtrage des données de fréquence cardiaque, si l'état de transition identifié est le troisième état de transition, en exécutant le troisième procédé, comprend les étapes ci-dessous consistant à :
vérifier si la valeur de fréquence cardiaque dans la deuxième fenêtre se situe entre une deuxième valeur de référence et une troisième valeur de référence, dans laquelle la deuxième valeur de référence est égale à la somme d'une valeur de fréquence cardiaque dans la deuxième fenêtre et de l'intervalle de tolérance de changement de fréquence cardiaque prédéfini, et la troisième valeur de référence est égale à la différence entre la valeur de fréquence cardiaque dans la deuxième fenêtre et l'intervalle de tolérance de changement de fréquence cardiaque prédéfini ;
si la valeur de fréquence cardiaque dans la deuxième fenêtre se situe entre la deuxième valeur de référence et la troisième valeur de référence, alors
réinitialiser la valeur de l'intervalle de tolérance afin qu'elle soit égale à une première valeur ; et
générer les données de fréquence cardiaque filtrées comme étant égales à la valeur de fréquence cardiaque dans la deuxième fenêtre ; et
si la valeur de fréquence cardiaque dans la deuxième fenêtre ne se situe pas entre la deuxième valeur de référence et la troisième valeur de référence :
générer les données de fréquence cardiaque filtrées comme étant égales à la valeur de fréquence cardiaque dans la première fenêtre ; et
augmenter la valeur de l'intervalle, de la première valeur.
